# NEUE EUROPÄISCHE PATENTSCHRIFT

(11) **EP 0 625 578 B2**
(45) Veröffentlichungstag und Bekanntmachung des Hinweises auf die Entscheidung über den Einspruch: **28.04.2004**
(45) Hinweis auf die Patenterteilung: 03.07.1996
(21) Anmeldenummer: 93120934.0
(22) Anmeldetag: 27.12.1993
(51) Int. Cl.: C12P 19/24, C12S 3/02, A23L 1/236, A23L 1/09, A23L 1/06, A23G 9/04, C07H 15/04, C07H 3/04

(54) **Süssungsmittel, Verfahren zur Herstellung desselben sowie dessen Verwendung**
Sweetener, process of preparation and use thereof
Edulcorant, son procédé de préparation et son utilisation

(30) Priorität: 06.05.1993 DE 4314961
(43) Veröffentlichungstag der Anmeldung: 23.11.1994
(73) Patentinhaber: SÜDZUCKER AKTIENGESELLSCHAFT MANNHEIM/OCHSENFURT, 68165 Mannheim (DE)
(72) Erfinder: Kunz, Markwart, Dr., D-67550 Worms (DE); Munir, Mohammad, Dr., D-67271 Kindenheim (DE); Degelmann, Hanspeter, Dipl.-Ing., D-67549 Worms (DE); Kowalczyk, Jörg, Dr. Dipl.-Chem., D-67269 Grünstadt (DE); Wach, Wolfgang, Dipl.-Chem., D-38118 Braunschweig (DE); Vogel, Manfred, Dr., D-67271 Neuleiningen (DE)
(74) Vertreter: Gleiss, Alf-Olav, Dr.jur. Dipl.-Ing.

(56) Entgegenhaltungen:
- EP-A- 0 109 009
- EP-A- 0 152 779
- EP-A- 0 483 755
- DE-A- 2 217 628
- FR-A- 2 179 966
- GB-A- 1 429 334
- ALIMENTA Bd. 19, Nr. 1 , 1980 Seiten 5 - 16 SCHIWECK 'Palatinit - Herstellung, technologische Eigenschaften und Analytik palatinithaltiger Lebensmittel'
- PATENT ABSTRACTS OF JAPAN vol. 9, no. 12 (C-261)(1735) 18. Januar 1985 & JP-A-59 162 953 (SHOWA DENKO K.K.) 13. September 1984
- CARBOHYDRATE RES. Bd. 164 , 1987 Seiten 477 - 485 MUNIR ET AL. '1-O-alpha-glucopyranosyl-D-fructose : Darstellung aus Saccharose und ihre Reduktion zu 1-O-alpha-D-glucopyranosyl-D-glucitol'

## Beschreibung

Die Erfindung betrifft ein neues Süßungsmittel, ein Verfahren zu dessen Herstellung sowie die Verwendung dieses Süßungsmittels in Nahrungs- und Genußmitteln.

Da Saccharose ein kalorienreiches Nahrungsmittel ist, Zahnkaries begünstigt und für Diabetiker ungeeignet ist, besteht ein Bedürfnis an anderen Süßungsmitteln, die im Gegensatz zu synthetischen Süßstoffen wie Saccharin, Cyclamat oder Aspartam keinen Nebengeschmack haben und körpergebende Eigenschaften besitzen.

Als Süßungsmittel wurden bislang unter anderem Maltit und Lactit sowie Isomaltit als nicht-kariogene, kalorienarme Süßungsmittel vorgeschlagen. Erstere sind wegen ihrer sirupartigen Konsistenz nur begrenzt einsetzbar, während letzteres bislang nicht auf wirtschaftliche Weise hergestellt werden konnte.

Isomaltit kann z.B. gemäß DE 2217 628 A1 über Isomaltulose als Zwischenstufe mit anschließender katalytischer Hydrierung erhalten werden. Die Ausbeute an der Zwischenstufe Isomaltulose beträgt nur 45 %, die Gesamtausbeute an Isomaltit liegt bei 41 %.

Man kann zwar gemäß EP 28 900 A1, EP 49 472 A1 und EP 91 063 A1 mit immobilisierten Bakterienzellen eine enzymatische Umwandlung der Saccharose zu Isomaltulose in einer Ausbeute von etwa 80 % ermöglichen, jedoch wird für die Herstellung von Isomaltit gereinigte Isomaltulose benötigt, so daß auch bei diesen Verfahren eine Ausbeuteminderung durch Kristallisation eintritt.

Darüber hinaus hat Isomaltit den Nachteil, daß es aufgrund seiner geringeren Löslichkeit zum Auskristallisieren in Lebensmitteln neigt, wodurch z.B. Schokolade einen sandigen Geschmack zeigt, Hartkaramellen trübe werden und sich in Marmeladen Kristalle bilden.

Es ist femer aus der DE 25 20 173 A1 bekannt, durch katalytische Reduktion von Isomaltulose in neutraler wässriger Lösung neben Isomaltit, also dem Glucopyranosyl-1,6-sorbit (= 1,6-GPS), auch den stereoisomeren Glucopyranosyl-1,6-mannit (= 1,6-GPM) bis zu einem Gewichtsverhältnis von 1 : 1 zu erhalten. Aufgrund seiner geringen Löslichkeit kann 1,6-GPM zwar leicht isoliert werden und ist als kalorienarmes, körpergebendes Produkt eine Bereicherung der Diätetik. Wegen seiner geringen Löslichkeit kristallisiert es aber noch leichter in den Lebensmitteln aus als Isomaltit und muß - da die Süßkraft nur etwa 40 % der von Saccharose beträgt - in höherer Menge zum Erreichen des gleichen Süßungseffektes eingesetzt werden.

Auch Abmischungen von 1,6-GPS bzw. 1,6-GPM mit anderen Zuckeralkoholen oder Zuckem ergeben keine befriedigenden Produkte. Selbst wenn man den zur Unterdrückung von Kristallisation bekannten Sorbit einsetzt, erhält man hygroskopische, also klebrige Produkte.

Letztlich ist in der EP 109 009 A1 ein Isomerisierungsprodukt, das mit *Protaminobacter rubrum* (CBS 574.77) aus Saccharose hergestellt wurde, mit der folgenden Zusammensetzung beschrieben:

| | |
|---|---|
| Fructose | 5 - 8 Gew.% a. TS-Gehalt |
| Glucose | 2 - 5 Gew.% a. TS-Gehalt |
| Saccharose | 0 - 0,5 Gew.% a TS-Gehalt |
| Isomaltulose | 65 - 72 Gew.% a. TS-Gehalt |
| Trehalulose | 10 - 20 Gew.% a TS-Gehalt |
| Oligomere | 3 - 6 Gew.% a. TS-Gehalt |

Ein solches Saccharidgemisch ist als diätetisches Süßungsmittel ungeeignet, da einige Komponenten kalorisch verwertet werden, insulinabhängigen Stoffwechsel zeigen und Zahnkaries fördern. Der Gehalt an Trehalulose läßt sich zwar auf bis zu 35 % steigern, wenn die Zuckermischung etwa 100 h mit freien oder trägerfixierten Bakterienzellen unter geeigneten Bedingungen gehalten wird. Wirtschaftlich ist dieses Verfahren jedoch nicht.

Der Erfindung liegt daher die Aufgabe zugrunde, ein Süßungsmittel bzw ein Verfahren zur Herstellung eines neuen kalorienarmen, nicht-kariogenen und für Diabetiker geeigneten Süßungsmittels vorzuschlagen, welches eine angenehme Süßwirkung mit guten körpergebenden Eigenschaften verbindet, in fester Form leicht und wirtschaftlich hergestellt werden kann und in den anwendungsgemäßen Konzentrationen nicht auskristallisiert.

Zur Lösung dieser Aufgabe wird daher einmal ein Verfahren zur Herstellung eines Süßungsmittels gemäß Hauptanspruch und zum anderen die nach diesem Verfahren hergestellten Süßungsmittel gemäß Anspruch 3 bis 5 und letztlich die Verwendung dieser Süßungsmittel für Nahrungs- und Genußmittel vorgeschlagen, wobei weitere Ausgestaltungen in den Unteransprüchen erwähnt sind.

Die Erfindung beruht auf der überraschenden Feststellung, daß man durch die Kombination der Verfahrensschritte von Isomerisierung der Saccharose, Entfernung von nicht isomerisierter Restsaccharose und katalytischer Hydrierung und vorzugsweise sowohl durch die entweder vor oder nach der Hydrierung vorgenommene chromatographische Behandlung und auch durch eine spezielle Auswahl von Bakterienstämmen Süßungsmittel mit den gewünschten Eigenschaften erhält.

Im folgenden wird die Erfindung näher erläutert, wobei die folgenden Kurzbezeichnungen verwendet werden:
1,6-GPS für 6-0-α-D-Glucopyranosyl-D-sorbit
1,1-GPS für 1-0-α-D-Glucopyranosyl-D-sorbit
1,1-GPM für 1-0-α-D-Glucopyranosyl-D-mannit
und wobei ferner festzuhalten ist, daß durch Hydrierung

| | | |
|---|---|---|
| aus Isomaltose | 100% | 1,6-GPS, |
| aus Isomaltulose | 43-57% | 1,1-GPM |
| | 43-57% | 1,6-GPS und |
| aus Trehalulose | 50-80% | 1,1-GPM |
| | 20-50% | 1,1-GPS |

erhalten werden.

Nach dem erfindungsgemäßen Verfahren wird in einer ersten Stufe Saccharose mit immobilisierten Bakterienstämmen aus der Gruppe von *Protaminobacter rubrum* (CBS 574.77), *Serratia plymuthica* (ATCC 15928), *Serratia marcescens* (NCIB 8285), *Leuconostoc mesenteroides (NRRL-B* 512 F (ATCC 1083 a)) und *Erwinia rhapontici* (NCPPB 1578) isomerisiert.

Danach wird in einem zweiten Verfahrensschritt diese "Isomerisierte Saccharose" von der nicht isomerisierten Restsaccharose befreit. Diese ist im vorliegenden Verfahren wesentlich, wenngleich es an sich bekannt ist, reine Saccharoselösungen mit Invertase und/oder einem Invertierungsharz zu einem Gemisch aus Glucose und Fructose zu spalten; nur war es bislang nicht bekannt, eine spezifische Spaltung der Saccharose in Gegenwart von wesentlich größeren Mengen anderer Disaccharide ohne deren Beeinträchtigung durchzuführen.

In einem dritten Verfahrensschritt wird dann die von Restsaccharose befreite "Isomerisierte Saccharose" katalytisch hydriert, wobei ein Gemisch der folgenden Zusammensetzung erhalten wird:

| | |
|---|---|
| Mannit (aus Fructose) | 3 bis 4 Gew.% |
| Sorbit (aus Fructose und Glucose) | 4 bis 9 Gew.% |
| 6-O-α-D-Glucopyranosyl-D-sorbit (= 1,6-GPS) aus Isomaltulose | 10 bis 55 Gew.% |
| 1-O-α-D-Glucopyranosyl-D-sorbit (= 1,1-GPS) aus Trehalulose | 2 bis 20 Gew.% |
| 1-O-α-D-Glucopyranosyl-D-mannit (= 1,1-GPM) aus Isomaltulose und Trehalulose | 30 bis 70 Gew.% |
| hydrierte Oligosaccharide | 3 bis 6 Gew.% |
| Saccharose | unter 1 Gew.% |

Das Verhältnis von GPS/GPM liegt je nach Hydrierungsbedingungen (alkalisch/neutral) bei etwa 2 : 1 bis 1 : 7.

Da die Oligosaccharide die anwendungstechnischen, aber auch die physiologischen Eigenschaften des erhaltenen Produktes negativ beeinflussen können, werden sie zusätzlich und vorzugsweise durch chromatographische Trennung an Kationenaustauscherharzen bzw. Zeolithen entfernt.

Das nach der chromatographischen Trennung resultierende Gemisch aus Sorbit, Mannit, 1,6-GPS, 1,1-GPS und 1,1-GPM kann in flüssiger Form oder auch als trockenes, freifließendes Produkt als Süßungsmittel verwendet werden.

Alternativ ist es also auch möglich, die isomerisierte Saccharose von der nicht hydrierfähigen Rest-Saccharose zu befreien und insbesondere Glucose, Fructose und Oligosaccharide zu entfemen, was durch chromatographische Trennung an Kationenaustauscherharzen bzw. Zeolithen bewirkt wird.

Um das Süßungsmittel besser in Lebensmitteln einzusetzen, bei denen ein hoher Trockensubstanzgehalt einzuhalten ist, ist es vorteilhaft, die Kristallisationsneigung des 1,1-GPM durch Erhöhung des 1,1-GPS Gehaltes zu unterdrücken.

Um das erhaltene Süßungsmittel, das als Gemisch aus Sorbit, Mannit, 1,6-GPS, 1,1-GPS und 1,1-G PM in flüssiger Form vorliegt in trockene Form zu überführen, muß das als Lösungsmittel vorliegende Wasser durch Verdampfen entfernt werden, wobei es vorteilhaft ist, zuvor den Sorbit- und Mannitgehalt auf 5 bis 0 und vorzugsweise auf 1 bis 0% zu reduzieren; dieses läßt sich durch die chromatographische Trennung an geeigneten Kationenaustauscherharzen bzw. Zeolithen durchführen.

Die erfindungsgemäß hergestellten Mischungen haben eine der Saccharose ähnliche Süße ohne Beigeschmack; die Süßkraft beträgt jedoch nur 40 bis 50 %. Diese kann gegebenenfalls durch Zufügen von synthetischen Süßstoffen erhöht und z.B. auf die Süßkraft von Saccharose eingestellt werden. Bei Anwendung in Karamellen oder Marmelade ergibt sich ein der Saccharose vergleichbarer Körper, ohne daß es zum Auskristallisieren der einzelnen Saccharide kommt.

### Beispiel 1:

### A. Herstellung des Biokatalysators

Von einer Abimpfung des Stammes *Protaminobacter rubrum* (CBS 574.77) werden Zellen mit 10 ml eines sterilen Nährsubstrates, bestehend aus 8 kg Dicksaft aus einer Zuckerfabrik (Trockensubstanzgehalt = 65 %), 2 kg Maisquellwasser, 0,1 kg (NH₄)₂ HPO₄ und 89,9 kg dest. Wasser, bei Bedarf auf pH 7,2 eingestellt, abgeschwemmt. Diese Suspension dient als Impfgut für die Schüttelmaschinen-Vorkultur in 1-Liter-Kolben mit 200 ml Nährlösung obiger Zusammensetzung.

Nach einer 30-stündigen Bebrütungszeit bei 29° C werden mit je 10 Kolben (Gesamtinhalt 2 Liter) 18 Liter Nährlösung obiger Zusammensetzung in einem 30-Liter-Kleinfermenter beimpft und bei 29° C mit 20 Liter Luft pro Minute und einer Rührgeschwindigkeit von 350 UpM fermentiert.

Nach Erreichen von Keimzahlen über 5 x 10⁹ Keimen/ml wird die Fermentation abgestellt; die Zellen werden durch Zentrifugation aus der Fermenterlösung abgeemtet, in einer 2%-igen Natriumalginatlösung suspendiert und durch Eintropfen der Suspension in eine 2%-ige Calciumchloridlösung immobilisiert.

Die entstandenen Immobilisatkugeln werden mit Wasser gewaschen. Dieser Biokatalysator ist bei +4° C mehrere Wochen lagerfähig.

### B. Herstellung der "Isomerisierten Saccharose"

Die wie unter A erhaltenen immobilisierten Zellen werden in einen temperierbaren Säulenreaktor gefüllt auf 25 bis 30° C temperiert und mit einer Saccharoselösung mit 35 bis 45 % TS-Gehalt kontinuierlich durchströmt. Die Fließgeschwindigkeit wird dabei so eingestellt, daß mindestens 97 % der eingesetzten Saccharose umgelagert werden.

Eine HPLC-Analyse der aus dem Säulenreaktor austretenden "Isomerisierten Saccharose" ergab folgende Zusammensetzung:

| | |
|---|---|
| Fructose | 2,5 % a. TS |
| Glucose | 2,0 % a. TS |
| Saccharose | 1,0 % a. TS |
| Isomaltulose | 82,5 % a. TS |
| Trehalulose | 9,5 % a. TS |
| Isomaltose | 1,5 % a. TS |
| Oligomere (DP > 3) | 1,0 % a. TS. |

### C. Entfernung von Restsaccharose

Die derart erhaltene "Isomerisierte Saccharose" wurde von der nicht hydrierfähigen Rest-Saccharose befreit, indem sie in einem mit H⁺-Ionen beladenen, starksauren Kationenaustauscher bzw. mit geeigneten Enzymen in einem Säulenreaktor wie folgt behandelt wurde:

### i) Entfernung der Restsaccharose an starksauren Kationenaustauschem

100 cm³ eines starksauren Kationenaustauschers (z.B. Lewatit^{(R)} OC 1052) wurden in eine geeignete, auf 60° C temperierte Glassäule eingefüllt und durch Regeneration mit HCl nach bekannter Methode mit H⁺-Ionen beladen.

Die nach Beispiel IB erhaltene "Isomerisierte Saccharose" wurde mit einer Fließrate von 100 cm³ · h⁻¹ durch die so vorbereitete Kationenaustauschersäule gepumpt. Das am Säulenausgang erhaltene Produkt hatte folgende Zusammensetzung (HPLC):

| | |
|---|---|
| Fructose | 3,0 % a. TS |
| Glucose | 2,5 % a. TS |
| Saccharose | - |
| Isomaltulose | 82,3 % a. TS |
| Trehalulose | 9,5 % a. TS |
| | |
| Isomaltose | 1,5 % a. TS |
| Oligomere (DP 3) | 1,2 % a. TS |

### ii) Entfernung der Restsaccharose durch Enzyme

11 g einer immobilisierten Invertase (z.B. SP 362 von NOVO Nordisk, Kophenhagen) entsprechend einem Bettvolumen von 33 cm³ wurden in eine geeignete, auf 60° C temperierte Glassäule eingefüllt.

Die nach Beispiel 1 B erhaltene "Isomerisierte Saccharose" wurde mit einer Fließrate von 210 cm³ h⁻¹ kontinuierlich durch diese Säule gepumpt.

Eine HPLC-Analyse des aus der "Invertasesäule" austretenden Produkts ergab folgende Zusammensetzung:

| | |
|---|---|
| Fructose | 3,0 % a. TS |
| Glucose | 2,5 % a. TS |
| Saccharose | - |
| Isomaltulose | 82,5 % a. TS |
| Trehalulose | 9,5 % a. TS |
| Isomaltose | 1,5 % a. TS |
| Oligomere (DP > 3) | 1,0 % a. TS |

In beiden Fällen wurde die Restsaccharose vollständig zu Glucose und Fructose gespalten. Der Gehalt an diesen Monosacchariden war entsprechend höher, während die anderen Komponenten der "Isomerisierten Saccharose" nicht verändert wurden.

### D Hydrierung der "Isomerisierten Saccharose"

Die jeweils von der Rest-Saccharose befreiten Ansätze der "Isomerisierten Saccharose" wurden an Raney-Nickel bei 80° C mit Wasserstoffgas unter Druck von etwa 10 MPa kontinuierlich hydriert. Nach Nickel-Abtrennung und Reinigung durch Ionenaustausch hatten die Ansätze der unter neutralen Bedingungen hydrierten "Isomerisierte Saccharose" etwa die folgende Zusammensetzung:

| | |
|---|---|
| Mannit | 1,5 % a. TS |
| Sorbit | 4,0 % a. TS |
| 1,6-GPS | 44,4 % a. TS |
| 1,1-GPS | 3,8 % a. TS |
| 1,1-GPM | 45,3 % a. TS |
| hydrierte und nichthydrierte Oligomere | 1,0 % a. TS |

Dieses Produkt ist zwar nach Entfernung von Wasser durch Verdampfen als Süßungsmittel einsetzbar, läßt sich jedoch wegen seiner Hygroskopizität, besonders wegen des Oligomerenanteils nur begrenzt einsetzen, zumal die noch vorhandenen Oligomeren teilweise im Dünndarm unter Freisetzung von Sorbit, Mannit und insbesondere von Glucose und Fructose gespalten werden und somit in Lebensmitteln für Diabetiker problematisch sind.

### Beispiel 2:

Es wurde analog Beispiel 1 A bis C "Isomerisierte Saccharose" hergestellt, die zur Entfemung von Glucose, Fructose und Oligosacchariden vorder Hydrierung einer chromatographischen Trennbehandlung unterworfen wurde, wobei gleichzeitig ein Verlust an den Disacchariden Isomaltulose, Isomaltose und Trehalulose vermieden werden sollte.

Als chromatographische Trennsäule wurde ein temperierbares, mit Siebboden versehenes 10 m langes Rohr mit 25 cm Durchmesser verwendet, das vollständig mit Wasser befüllt und anschließend mit einem mit Calciumionen beladenen starksauren Kationenaustauscherharz mit einer 4 bis 6%igen Vernetzung und einer Komgröße von etwa 0,4 bis 0,5 mm derart beschickt war, daß das Harz vollständig von Wasser bedeckt war.

Die nach Beispiel 1 A und B erhaltene "Isomerisierte Saccharose" wurde nach Entfernung der Restsaccharose in einer Menge von etwa 18 kg (Trockensubstanz) auf die auf etwa 75° C temperierte Trennsäule aufgetragen und mit entionisiertem Wasser mit einer Fließgeschwindigkeit von etwa 2 cm/min eluiert. Am Ausgang der Trennsäule wurden alle 10 Minuten Fraktionen gesammelt und deren Zusammensetzung mit HPLC untersucht.

In den ersten vier Fraktionen befanden sich etwa 60 % der Oligosaccharide und ferner etwa 10 % der Isomaltulose und etwa 25 % der Isomaltose. Die letzten fünf Fraktionen enthielten etwa 70 % der Fructose, 10 % der Trehalulose und 20 % der Glucose.

Die Zusammensetzung der resultierenden "Isomerisierten Saccharose" war wie folgt:

| | |
|---|---|
| Fructose | 1,0 % a. TS |
| Glucose | 2,3 % a. TS |
| Isomaltulose | 85,1 % a. TS |
| Trehalulose | 9,8 % a. TS |
| Isomaltose | 1,3 % a. TS |
| Oligomeren | 0,5 % a. TS |

Durch diese Art der Trennung lassen sich immerhin etwa 60 % der Oligomeren, 70 % der Fructose und 20 % der Glucose entfernen, wobei man den Verlust von 10 % der Isomaltulose, 10 % der Trehalulose und 25 % der Isomaltose hinnehmen muß.

Das erhaltene Produkt wurde analog Beispiel 1 C hydriert und hatte folgende Zusammensetzung:

| | |
|---|---|
| Mannit | 0,5 % a. TS |
| Sorbit | 3,3 % a. TS |
| 1,6-GPS | 43,8 % a. TS |
| 1,1-GPS | 3,9 % a. TS |
| 1,1-GPM | 48,5 % a. TS |
| Oligomere | 0,5 % a. TS. |

Gegenüber dem Produkt nach Beispiel 1 lag der Gehalt an Sorbit und Mannit niedriger; der Anteil an hydrierten und nicht hydrierten Oligomeren betrug nur die Hälfte.

### Beispiel 3:

Es wurde analog Beispiel 2 gearbeitet, wobei jedoch jetzt die Trennsäule mit einem Zeolithen beschickt wurde, der ein Si/Al-Verhältnis von etwa 50 hatte.

Die ersten fünf Fraktionen enthielten die gesamte Menge an Oligosacchariden, Glucose und Fructose und noch etwa 50 % der Isomaltose. Es traten weder Trehalulose noch Isomaltulose in diesen Fraktionen auf. Da der Isomaltosegehalt der "Isomerisierten Saccharose" nach Beispiel 1 bzw. 2 bei 1,5 bzw. 1,3 % a TS liegt, gehen bei dieser Arbeitsweise nur etwa 0,8 bzw. 0,5 % der gewünschten Disaccharide verloren. Gleichzeitig werden die unerwünschten Oligosaccharide, Glucose und Fructose vollständig entfernt.

Die erhaltene "Isomerisierte Saccharose" hatte die folgende Zusammensetzung:

| | |
|---|---|
| Fructose | - |
| Glucose | - |
| Isomaltulose | 89,0 % a. TS |
| Trehalulose | 10,2 % a. TS |
| Isomaltose | 0,8 % a. TS |
| Oligomere | - |

Dieses Produkt wurde analog Beispiel 1 D hydriert und zeigte folgende Zusammensetzung:

| | |
|---|---|
| 1,6-GPS | 45,3 % a. TS |
| 1,1-GPS | 4,1 % a. TS |
| 1,1-GPM | 50.6 % a. TS. |

Dieses von Mannit, Sorbit und Oligomeren befreite Produkt war ein ausgezeichnetes, kaum hygroskopisches und für Diabetiker geeignetes Süßungsmittel.

### Beispiel 4:

Die nach Beispiel 1 erhaltene und bereits hydrierte "Isomerisierte Saccharose" wurde mit einer chromatographischen Trennsäule, wie sie in Beispiel 2 vor der Hydrierung verwendet wurde, behandelt, wobei analog etwa 18 kg a. TS der nunmehr hydrierten "Isomerisierten Saccharose" aufgegeben und mit einer Fließgeschwindigkeit von 2 cm/min eluiert wurden. Allerdings war diese Trennsäule jetzt mit einem mit Natriumionen beladenen, starksauren Kationenaustauscherharz beschickt.

Die ersten drei Fraktionen enthielten die Oligomeren und etwa 4 % des 1,1-GPM. In den weiteren Fraktionen 4 bis 8 war der restliche Anteil an 1,1-GPM, der gesamte Anteil an 1,1-GPS und etwa 99 % des 1,6-GPS enthalten, sowie etwa 50 % des Mannits und nur geringe Anteile des Sorbits. Aus den Massenbilanzen ergibt sich für das Produkt der Fraktionen 4 bis 8 ein Gesamtgehalt von GPM und GPS von etwa 97 Gew.%. Die restlichen Anteile an 1,6 GPS, Mannit und Sorbit wurden in den Fraktionen ab Fraktion 9 eluiert.

Im Vergleich mit dem Verfahren gemäß Beispiel 2, nach welchem die chromatographische Trennung mit erdalkalibeladenen Kationenaustauschern durchgeführt wurde, ergibt sich eine bessere Trennung zwischen Disaccharid- und Monosaccharidalkoholen, so daß mehr als 97 Gew.% der gewünschten Disaccharidalkohole im Hauptprodukt gewonnen werden können, während bei mit Calciumionen beladenen Kationenaustauschem die Ausbeute bei etwa 85 % liegt. Überraschenderweise ergibt sich auch noch der weitere Vorteil, daß mehr als 90 % des als verwertbares Nebenprodukt entstandenen Sorbits mit einer Reinheit von mehr 98 % gewonnen werden kann.

Die Produktzusammensetzung war wie folgt:

| | |
|---|---|
| 1,6-GPS | 46,2 % a. TS |
| 1,1-GPS | 4,1 % a TS |
| 1,1-GPM | 49,6 % a. TS. |

### Beispiel 5:

Es wurde analog Beispiel eine chromatographische Trennung nach der Hydrierung durchgeführt, wobei jedoch jetzt eine Zeolith-Trennanlage entsprechend Beispiel 3 eingesetzt wurde. Hydrierte "Isomerisierte Saccharose" wurde in einer Menge von 15 bis 20 kg (Trockensubstanz) aufgetragen und mit entionisiertem Wasser eluiert.

Die Analyse der erhaltenen Fraktionen zeigt, daß Mannit, Sorbit und Oligomere sich vollständig in den ersten 5 Fraktionen befinden. Außerdem sind darin auch etwa 5 % des GPM-Anteils enthalten. Der restliche GPM, der gesamte 1,1-GPS sowie 1,6-GPS finden sich in den Fraktionen 6 bis 16 wieder.

Somit gelingt es, mehr als 97 % der erwünschten Disaccharidalkohole frei von Sorbit, Mannit und Oligomeren zu gewinnen.

### Beispiel 6:

Zur Kristallisation der die Disaccharidalkohole enthaltenden Fraktionen wird ganz allgemein der Wassergehalt durch Verdampfen entfernt. Hierzu wurden diese Fraktionen z.B. die nach Beispiel 5 erhaltene Fraktion durch Verdampfen unter vermindertem Druckauf 90 bis 95 % Trockensubstanzgehalt aufkonzentriert, auf einer gekühlten Fläche erstarren gelassen und anschließend gemahlen. Es wurde ein feinkörniges, nicht klebendes und freifließendes Produkt erhalten.

Wenn das Süßungsmittel in Lebensmitteln mit einem hohen Trockensubstanzgehalt eingesetzt werden soll, ist es von Vorteil, die Kristallisationsneigung des 1,1-GPM durch Erhöhung des 1,1-GPS zu unterdrücken.

### Beispiel 7:

Zur Feststellung der relativen Süßkraft wurden in einem Dreieckstest von jeweils 15 Versuchspersonen folgende Lösungen miteinander verglichen:
a) Zwei 7 %ige Saccharoselösungen gegen eine 15,5 %ige Lösung des neuen Süßungsmittels gemäß Beispiel 3.
b) Zwei 7 %ige Saccharoselösungen gegen eine 17,5 %ige Lösung dieses neuen Süßungsmittels.
c) Zwei 7 %ige Saccharoselösungen gegen eine 18,5 %ige Lösung dieses neuen Süßungsmittels.

Beim Test a) wurde von sechs Personen das neue Süßungsmittel herausgefunden: Kein statistisch gesicherter Unterschied zu den Saccharoselösungen.

Beim Test b) wurde von zwölf Personen das neue Süßungsmittel als "süßer" herausgefunden: Statistisch gesicherter Unterschied mit p = 0,99.

Beim Test c) wurde ebenfalls von zwölf Personen das neue Süßungsmittel als "süßer" herausgefunden: Statistisch gesicherter Unterschied mit p = 0,99.

Die Süßkraft des erfindungsgemäßen Süßungsmittels beträgt 45 % der Saccharose. Zur Anhebung der Süßkraft läßt sich das neue Süßungsmittel mit Fructose, Xylit, Saccharin, Cyclamat, Aspartam oder Acesulfam-K mischen.

### Beispiel 8:

Zur Herstellung von Speiseeis mit dem neuen Süßungsmittel wurden 22,1 kg süße Sahne (40 % Fett i. Trockenmasse), 58,1 kg Vollmilch (3,7 % Fett i.T.) und 4,5 kg Magermilchpulver mit 15 kg des Süßungsmittels gemäß Beispiel 3 und 0,3 kg Stabilisator vermischt, homogenisiert und sterilisiert. Nach der Sterilisation wurden 53 g fein gemahlener Phenylalaninasparaginsäuremethylester der Eismasse zugesetzt, verrührt, aufgeschlagen und gefroren. Das Produkt hat die gleiche Süße und den gleichen Geschmack, wie mit 15 kg Zucker hergestelltes Speiseeis.

Bei Frucht-Eiskrem ist es sogar von Vorteil, auf eine Aufsüßung zu verzichten, da das neue Süßungsmittel den Fruchtgeschmack wesentlich besser zur Geltung bringt.

### Beispiel 9.

Zur Herstellung einer kalorienarmen Erdbeerkonfitüre wurden 1 kg zerkleinerte Erdbeeren zusammen mit 1 kg des neuen Süßungsmittels gemäß Beispiel 3 und 8 g eines mittelveresterten Pektins mit 150° SAG-USA (Ullmann, Enzyklopädie der technischen Chemie, 3. Auflage, Bd. 13, S. 180) sowie 7 g Weinsäure, drei Minuten lang gekocht und in vorbereitete Gläser abgefüllt.

Ein Vergleich mit einer mit Zucker hergestellten Konfitüre zeigte keinen Unterschied bezüglich Konsistenz, die Süße war etwas geringer, dafür jedoch der Erdbeergeschmack spürbar stärker. Nach Lagerung von sechs Monaten Dauer zeigte sich keine Kristallisationsneigung des Süßungsmittels.

## Patentansprüche

1. Verfahren zur Herstellung eines Süßungsmittels mit einem Gehalt an 6-O-α-D-Glucopyranosyl-D-sorbit (=1,6-GPS), 1-O-α-D-Glucopyranosyl-D-sorbit (=1,1-GPS) und 1-O-α-D-Glucopyranosyl-D-mannit (=1,1-GPM) durch enzymatische Umwandlung von Saccharose in ein vorwiegend Isomaltulose enthaltendes Produkt und spätere Hydrierung zu 1,6-GPS, 1,1-GPM und 1,1-GPS enthaltenden Produkten, **dadurch gekennzeichnet, dass** man
a) in einem ersten Verfahrensschritt Saccharose enzymatisch unter Einsatz von immobilisierten Zellen von Bakterienstämmen aus der Gruppe von Protaminobacter rubrum (CBS 574.77), Serratia plymuthica (ATCC 15928), Serratia marcescens (NCIB 8285), Leuconostoc mesenteroides (NRRL-B 512 F(ATCC 1083a))und Erwinia rhapontici (NCPPB 1578) in ein als "isomerisierte Saccharose" bezeichnetes, Trehalulose und Isomaltulose enthaltendes Saccharidengemisch mit einem Disaccharidanteil von mehr als 85 Gew.-% umwandelt,
b) in einem zweiten Schritt die "isomerisierte Saccharose" von nicht isomerisierter Restsaccharose durch enzymatische und/oder H⁺ katalysierte Spaltung befreit,
c) in einem weiteren Verfahrensschritt die "isomerisierte Saccharose" katalytisch hydriert, wobei vorzugsweise
d) entweder vor oder nach der katalytischen Hydrierung das erhaltene Gemisch einer chromatographischen Trennung unterworfen wird,
e) und das Süßungsmittel isoliert, das 10 bis 50 Gew.-% 1,6-GPS, 2 bis 20 Gew.-% 1,1-GPS und 30 bis 70 Gew.-% 1,1-GPM enthält.

2. Verfahren nach Anspruch 1, **dadurch gekennzeichnet, dass** man die chromatographische Trennung zur Entfernung der in der Mischung enthaltenen Oligosaccharidalkohole und/oder Monosaccharidalkohole an mit Natrium-, Kalium- oder Calcium-Ionen beladenen, starksauren Kationenaustauscherharzen oder an Zeolithen mit einem Si/Al-Verhältnis >50 durchführt.

3. Süßungsmittel mit einem Gehalt an 1,6-GPS, 1,1-GPS und 1,1-GPM, hergestellt nach einem Verfahren gemäß der Ansprüche 1 oder 2, **dadurch gekennzeichnet, dass** das Süßungsmittel 10 bis 50 Gew.-% 1,6-GPS, 2 bis 20 Gew.-% 1,1-GPS und 30 bis 70 Gew.-% 1,1-GPM enthält.

4. Süßungsmittel nach Anspruch 3, **dadurch gekennzeichnet, dass** in dem Süßungsmittel 25 bis 50 Gew.-% 1,6-GPS, 2 bis 20 Gew.-% 1,1-GPS und 35 bis 60 Gew.-% 1,1-GPM enthalten sind.

5. Süßungsmittel nach einem der Ansprüche 3 oder 4, **dadurch gekennzeichnet, dass** es geringe Mengen Mannit, Sorbit, hydrierte oder nicht hydrierte Oligosaccharide (Polymerisationsgrad DP>3) oder Gemische dieser enthält.

6. Verwendung von Süßungsmitteln nach einem der Ansprüche 3, 4 oder 5 in fester oder flüssiger Form als Süßungsmittel für Nahrungs- und Genussmittel.

## Claims

1. Method for producing a sweetening agent having a content of 6-O-α-D-glucopyranosyl-D-sorbitol (=1,6-GPS), 1-O-α-D-glucopyranosyl-D-sorbitol (=1,1-GPS) and 1-O-α-D-glucopyranosyl-D-mannitol (=1,1-GPM) by enzymatically converting saccharose into a product containing mainly isomaltulose, and later hydrogenation to form products containing 1,6-GPS, 1,1-GPM and 1,1-GPS **characterised in that**
a) in a first method step, saccharose is enzymatically converted using immobilised cells of bacteria strains from the group of Protaminobacter rubrum (CBS 574.77), Serratia plymuthica (ATCC 15928), Serratia marcescens (NCIB 8285), Leuconostoc mesenteroides (NRRL-B 512 F(ATCC 1083a)) and Erwinia rhapontici (NCPPB 1578) into a saccharide mixture, which is termed "isomerised saccharose" and contains trehalulose and isomaltulose, having a disaccharide portion of more than 85% by weight,
b) in a second step the "isomerised saccharose" is freed from non-isomerised remaining saccharose by enzymatic and/or H⁺-catalysed dissociation,
c) in a further method step the "isomerised saccharose" is catalytically hydrogenated, in which case preferably
d) either before or after the catalytic hydrogenation, the mixture which is obtained is subjected to chromatographic separation,
e) and the sweetening agent is isolated which contains 10 to 50% by weight 1,6-GPS, 2 to 20% by weight 1,1-GPS and 30 to 70% by weight 1,1-GPM.

2. Method according to claim 1, **characterised in that** the chromatograhic separation for removing the oligosaccharide alcohols and/or monosaccharide alcohols contained in the mixture is carried out on strongly acidic cationic exchanger resins charged with sodium ions, potassium ions or calcium ions, or on zeolites having a Si/Al ratio of >50.

3. Sweetening agent having a content of 1,6-GPS, 1,1-GPS and 1,1-GPM, produced according to a method in accordance with claims 1 or 2, **characterised in that** it contains
10 to 50% by weight 1,6-GPS,
2 to 20% by weight 1,1-GPS and
30 to 70% by weight 1,1-GPM.

4. Sweetening agent according to claim 3, **characterised in that** it contains
25 to 50% by weight 1,6-GPS,
2 to 20% by weight 1,1-GPS,
35 to 60% by weight 1,1-GPM.

5. Sweetening agent according to one of the claims 3 or 4 **characterised in that** it contains small amounts of mannitol, sorbitol, hydrogenated or non-hydrogenated oligosaccharide (degree of polymerisation DP > 3) or mixtures of these.

6. Use of sweetening agents according to one of the claims 3,4 or 5 in solid or liquid form as a sweetening agent for foodstuffs and luxury foods.

## Revendications

1. Procédé de fabrication d'un édulcorant ayant une teneur en 6-O-α-D-glucopyranosyl-D-sorbite (=1,6-GPS), 1-O-α-D-glucopyranosyl-D-sorbite (=1,1-GPS) et 1-O-α-D-glucopyranosyl-D-mannite (=1,1-GPM), par conversion enzymatique de la saccharose en un produit contenant principalement de l'isomaltulose et hydrogénation ultérieure pour donner des produits contenant du 1,6 GPS, de la 1,1 GPM et du 1,1 GPS, **caractérisé en ce que**
a) lors d'une première étape du procédé, on convertit la saccharose par voie enzymatique en utilisant des cellules immobilisées des souches bactériennes issues du groupe de Protaminobacter rubrum (CBS 574.77), Serratia plymuthica (ATCC 15928), Serratia marcescens (NCIB 8285), Leuconostoc mesenteroides (NRRL-B 512 F(ATCC 1083a)) et Erwinia rhapontici (NCPPB 1578) en un mélange de saccharides contenant de la tréhalulose et de l'isomaltulose, dénommé "saccharose isomérisée" ayant une proportion de disaccharides supérieure à 85% en poids,
b) lors d'une deuxième étape, la "saccharose isomérisée" est débarrassée de la saccharose restante non isomérisée par dissociation enzymatique et/ou catalysée par H⁺,
c) lors d'une étape ultérieure du procédé, la "saccharose isomérisée" est hydrogénée de façon catalytique, où de préférence
d) de mélange obtenu est soumis soit avant soit après hydrogénation catalytique, à une séparation chromatographique,
e) et l'édulcorant est isolé contenant de 10 à 50% en poids de 1,6 GPS, de 2 à 20% en poids de 1,1 GPS et de 30 à 70% en poids de 1,1 GPM.

2. Procédé selon la revendication 1, **caractérisé en ce que** l'on effectue la séparation chromatographique visant à éliminer les alcools d'oligosacccharides contenus dans le mélange et/ou les alcools de monosaccharides sur des résines échangeuses de cations chargées en ions sodium, potassium ou calcium, fortement acides, ou bien sur des zéolithes ayant un rapport Si/Al >50.

3. Edulcorant ayant une teneur en 1,6 GPS, 1,1 GPS et 1,1 GPM, fabriqué suivant un procédé selon les revendications 1 ou 2, **caractérisé en ce qu'**il contient
de 10 à 50% en poids de 1,6 GPS,
de 2 à 20% en poids de 1,1 GPS et
de 30 à 70% en poids de 1,1 GPM.

4. Edulcorant selon la revendication 3, **caractérisé en ce qu'**il contient
de 25 à 50% en poids de 1,6 GPS,
de 2 à 20% en poids de 1,1 GPS,
de 35 à 60% en poids de 1,1 GPM.

5. Edulcorant selon l'une des revendications 3 ou 4, **caractérisé en ce qu'**il contient de faibles quantités de mannite, sorbite, d'oligosaccharides hydrogénées ou non hydrogénées (degré de polymérisation DP > 3) ou bien des mélanges de ces produits.

6. Utilisation d'édulcorant selon l'une des revendications 3, 4 ou 5, sous forme solide ou liquide comme édulcorant pour des produits alimentaires et de consommation.
